# FASCICULE DE BREVET EUROPEEN

(11) **EP 1 106 172 B1**
(45) Date de publication et mention de la délivrance du brevet: **21.06.2006**
(21) Numéro de dépôt: 99403056.7
(22) Date de dépôt: 07.12.1999
(51) Int. Cl.: A61K 8/02, A61K 8/92, A61Q 1/14

(54) **Produit tel qu'un tampon à démaquiller comprenant une face externe destinée à appliquer des produits aqueux sur la peau**
Artikel, insbesondere eine Watte mit einer Oberfläche um wässrige Produkte auf die Haut aufzutragen
Pad like article for removing make-up comprising one external surface for applying aqueous products on the skin

(43) Date de publication de la demande: 13.06.2001
(73) Titulaire: Georgia-Pacific France, 68320 Kunheim (FR)
(72) Inventeur: Gregoire, Philippe, 27700 Les Andelys (FR)
(74) Mandataire: Cortier, Sophie

(56) Documents cités:
- EP-A- 0 595 683
- EP-A- 0 870 496
- EP-A- 0 879 575
- DE-A- 2 119 819
- FR-A- 2 649 318
- US-A- 5 968 025
- DATABASE WPI Section Ch, Week 198842 Derwent Publications Ltd., London, GB; Class A96, AN 1988-296046 XP002139176 & JP 63 215800 A (KAO CORP), 8 septembre 1988 (1988-09-08)

## Description

L'invention concerne un produit de coton destiné aux soins de la peau, tel qu'un tampon à démaquiller, comprenant une face externe destinée à appliquer des produits aqueux sur la peau.

Ce produit de coton est un produit ou tampon découpé en formats, de forme ronde communément appelé "disque à démaquiller", ovale, carré ou toute autre forme.

Dans le texte qui suit, le terme "tampon à base de fibres de coton hydrophiles" englobe tout produit comprenant essentiellement des fibres de coton hydrophiles et absorbantes dans une proportion allant de 70 à 100 pour cent de fibres de coton et de 0 à 30 pour cent de fibres artificielles telles que des fibres de viscose ou de fibres synthétiques telles que des fibres de polyoléfines ou des fibres bicomposantes (par exemple polyester/polyester, polyester/ polypropylène, polypropylène/polyéthylène).

Le produit de coton a un grammage compris dans l'intervalle allant de 150 à 400 g/m², de préférence allant de 180 à 300 g/m².

Les soins de la peau comprennent les soins du corps, les soins du visage et plus particulièrement les soins de beauté utilisant des produits cosmétiques : le démaquillage et le maquillage du visage, les soins de bébé : toilette et change du bébé, etc.

Du fait du caractère hydrophile des fibres de coton, un inconvénient majeur des produits de coton ou tampon est leur trop grande capacité d'absorption des produits appliqués sur leur surface. En effet, le produit généralement aqueux pénètre à l'intérieur du tampon de coton par capillarité, parfois même traverse le tampon et imprègne les mains.

Le produit de soin utilisé est gaspillé et le tampon se déforme, étant imprégné de produit.

On a trouvé des solutions au problème de traversée totale du tampon par le produit de soin.

Une des solutions par exemple décrite dans le brevet américain N° 5 230 119 consiste à fournir un tampon muni d'une poignée et d'une couche arrière constituée d'un film plastique fin imperméable ou d'une feuille de papier traitée imperméable.

Une autre solution décrite dans le brevet européen n° 0 441 667 consiste à utiliser pour le démaquillage un tampon à démaquiller comprenant une couche d'arrêt centrale hydrophobe et/ou étanche vis à vis du produit à démaquiller et de part et d'autre de laquelle adhèrent deux couches de matériau absorbant. Le matériau absorbant utilisé est de préférence du coton peigné ou cardé. La couche d'arrêt peut être constituée par une pellicule formée d'une ou plusieurs matières plastiques souples, telles que par exemple du polyéthylène, du polypropylène ou du polyester.

Mais dans ces solutions, les couches imperméables ou couches d'arrêt sont rapportées, elles ne sont pas constituées du matériau absorbant ou de la nappe formant le tampon. Elles sont totalement imperméables et ne peuvent pas être destinées au contact de la peau.

En jouant sur la nature des fibres, il est également possible de modifier les propriétés du tampon de coton ou encore de l'une de ses couches extérieures.

Par exemple, on peut incorporer en mélange avec des fibres de coton hydrophiles, des fibres hydrophobes polyoléfiniques se prêtant au cardage. De telles fibres sont décrites dans le brevet européen n° 0 687 318. Mais on modifie alors la composition fibreuse du produit de coton.

Sans modifier la composition ni la structure du produit, le brevet américain n° 5 480 699 décrit des moyens mécaniques de compression pour éviter la pénétration de grosses quantités de crèmes ou liquides dans le tampon. En effet, en comprimant l'une des couches extérieures constituées de voile de carde, de manière plus importante, et en associant les deux voiles de carde à une couche centrale non comprimée, le produit final présente une surface plus comprimée dans laquelle les fibres sont resserrées et laissent moins d'interstices pour le passage des crèmes ou liquides à appliquer sur la peau. Mais cette solution n'empêche pas une certaine quantité de liquide ou crème de pénétrer à l'intérieur du tampon par les interstices restants, la compression améliorant la capillarité. Sur ce type de produit, l'absorption n'est pas retardée.

Le problème posé dans la présente invention est de retarder l'absorption des produits aqueux sur une des faces du produit de coton sans rendre imperméable cette même face de manière définitive et sans modifier la composition fibreuse du produit de coton.

Il s'agit en fait d'une "imperméabilisation" temporaire qui a pour fonction d'empêcher la pénétration de produits de soin au moment de leur application sur les tampons de coton et de retarder l'absorption, permettant ainsi l'application des produits de soin sur la peau avant qu'ils n'aient le temps de pénétrer à l'intérieur du tampon de coton.

L'invention a pour but de fournir un produit de coton tel qu'un tampon à démaquiller, à base de fibres de coton hydrophiles, apportant une solution au problème posé ci-dessus pour appliquer efficacement des produits aqueux sur la peau.

Selon une caractéristique essentielle de l'invention, une des faces externes du produit de coton comprend un agent retardant l'absorption des produits aqueux.

Ledit l'agent retardant l'absorption des produits aqueux

est une émulsion de cire naturelle d'origine minérale, végétale ou animale.

Selon une caractéristique particulièrement préférée de invention, l'agent est une émulsion de cire d'abeille.

Selon une autre caractéristique de l'invention, l'agent est une émulsion ou dispersion comprenant au moins 30 % de matières actives.

Selon une autre caractéristique avantageuse de invention, la première face comprend au moins 0,3 g/m² de matières actives.

D'autres caractéristiques et avantages de invention apparaîtront plus en détails dans la description qui suit.

Dans le but d'optimiser l'application d'un produit de soin sur la peau ou encore efficacité du nettoyage de la peau, du démaquillage et/ou du maquillage à l'aide de produits cosmétiques, le produit ou tampon de coton selon l'invention comprend un agent retardant l'absorption de produits aqueux sur une de ses faces externes que l'on qualifiera ici de première face externe.

Appliqués en faible quantités sur des produits de coton classiquement hydrophiles et absorbants, on a pu observer de manière surprenante que ces composants permettaient de retarder l'absorption des produits aqueux déposés en surface des produits de coton.

Le composant retardant l'absorption de produits aqueux est une émulsion de cire naturelle, d'origine minérale, végétale ou animale.

Des exemples de cire d'origine animale sont la cire de spermaceti et la cire d'abeille.

Des exemples de cire d'origine végétale sont la cire de candellila et la cire de camauba.

Des exemples de cire d'origine minérale sont la cérésine et l'azocérite.

L'émulsion de cire d'abeille est particulièrement avantageuse et appropriée pour l'usage cosmétique du tampon, c'est un composant testé dermatologiquement. Il s'agit d'une émulsion cationique de cire d'abeille blanchie qui comprend de la cire d'abeille, de l'eau, des agents émulsifiants, glycéryl stéarate et diéthanolaminoéther stéarate.

La cire d'abeille elle-même est composée d'esters d'acides gras cireux tels que du myricyl palmitate, d'acide cérotique et d'autres acides cireux homologues et de petites quantités d'hydrocarbones, d'esters de cholestérol et d'alcools céryliques.

L'agent retardant l'absorption est une émulsion ou une dispersion comprenant au moins 30 % de matières actives.

La première face du produit ou tampon de coton selon l'invention comprend au moins 1 g/m² d'émulsion appliquée, c'est-à-dire au moins 0,3 g/m² de matières actives déposées.

La première face comprenant en surface un tel agent acquiert des propriétés très avantageuses.

Elle retarde la pénétration des produits aqueux appliqués en surface des tampons.

La pénétration des produits de soin tels que laits de toilette, produits démaquillants ou de démaquillage est un inconvénient majeur des tampons en coton hydrophile classiques. Les produits de soin ou produits cosmétiques sont gaspillés et ne sont pas utilisés de manière économique, ils traversent parfois le tampon. L'efficacité du nettoyage de la peau n'est pas optimisée.

Avec un tel traitement de la première face du tampon, une "imperméabilité" temporaire à l'eau est créée, ceci permet d'éviter l'absorption quasi spontanée des produits aqueux par les fibres de coton hydrophile dès leur dépôt sur le tampon.

Un test simple mettant en évidence cette propriété consiste à déposer à température ambiante (environ 20°C) en surface d'un récipient rempli d'eau, des tampons ainsi traités selon l'invention, la face traitée étant tournée vers l'extérieur et la face absorbante vers l'eau, et des tampons de l'état de la technique constitués de cent pour cent de fibres de coton. Les premiers restent en surface pendant au moins 5 minutes, les autres s'imprègnent quasiment instantanément d'eau et sont très rapidement immergés, en général au bout de 3 à 5 secondes.

L'avantage ainsi procuré est de pouvoir garder les produits en surface plus longtemps et d'utiliser la quantité totale de produit déposé sur le tampon pour les soins de la peau, sans perte de produit et sans déformation du tampon.

Un test d'usage interne à la société demanderesse a été réalisé par 25 personnes utilisant habituellement et exclusivement des tampons B de coton hydrophile appartenant à l'état de la technique, pour les soins de la peau, le démaquillage, etc...

Les tampons B constitués de cent pour cent de fibres de coton, sont issus de nappes de coton fabriquées suivant la demande de brevet européen n° 0 735 175 et sont commercialisés sous la marque DEMAK'UP® .

Des tampons A selon l'invention, dont la première face a été traitée par un agent retardant l'absorption, plus précisément une émulsion de cire d'abeille, ont été testés comparativement aux tampons B de l'état de la technique.

Les observations suivantes ont été relevées.

Pour les tampons A selon l'invention dont la première face a été traitée, presque la totalité des personnes : 92 %, a noté la capacité du tampon à retarder l'absorption des produits de soin déposés en surface des tampons.

Avec les tampons selon l'invention dont la première face a été traitée, le nombre de personnes ayant trouvé une amélioration du nettoyage de la peau, s'élève à 92 %.

Enfin, pour le démaquillage, 85 % des personnes ont observé une meilleure efficacité du démaquillage avec des tampons selon l'invention avec traitement de la première face.

Des avantages similaires ont pu être observés lors de l'utilisation de produits de maquillage, tels que des lotions, de crèmes, des fonds de teint, des fards à joue, pour appliquer et répartir le produit sur la peau.

Les personnes ont également utilisé avantageusement le produit selon l'invention, pour appliquer des produits de parfumerie tels que des eaux de toilette. L'absorption immédiate de l'eau de toilette par le tampon est évitée comparativement à l'application d'eau de toilette avec des produits de coton de l'art antérieur.

Le produit ou tampon de coton selon l'invention est constitué d'une ou plusieurs nappes de fibres de coton obtenues par nappage, par cardage ou par voie pneumatique.

Ces nappes peuvent être de qualité fibreuse identique ou différente. Elles peuvent être constituées directement à partir de coton hydrophile et blanchi. Elles peuvent aussi être obtenues à partir de coton brut écru, puis traitées chimiquement. Elles sont ensuite superposées et associées par tout moyen connu tels que des moyens de collage ou des moyens mécaniques comme le calandrage ou l'aiguilletage. Les moyens d'association peuvent encore être hydrauliques.

On peut obtenir une bonne association par imprégnation, pulvérisation, ou déversement d'une solution. Cette imprégnation est associée à un exprimage compactant la nappe et éliminant une partie de la quantité de liquide contenu dans la nappe humide par exemple par calandrage ou passage sur une fente à vide.

La technique d'hydroliage permet à la fois d'associer les les deux nappes entre elles et de lier les surfaces de la nappe.

Le traitement par hydroliage peut intervenir dans le cas d'une nappe écrue qui va être traitée chimiquement, juste après l'étape d'imprégnation de la nappe comme cela est décrit dans la demande de brevet européen n° 0 735 175.

Cette étape d'hydroliage peut aussi être placée en phase finale de rinçage suivant le brevet européen n° 0 805 888 au nom de la demanderesse où la fabrication de la nappe de coton se fait en ligne continue.

Lors de la fabrication du produit selon l'invention, une des faces externes (première face externe) du produit de coton est traitée de manière à retarder l'absorption des produits aqueux (produits de soins, ...) pour un usage cosmétique des tampons.

Après les étapes d'imprégnation, et avant ou après séchage, on traite cette première face en appliquant un agent retardant l'absorption de produits aqueux, précédemment décrit. Suivant un mode préféré de l'invention, on applique une émulsion de cire dans une quantité d'au moins 1 g/m², ce qui revient à déposer au moins 0,3 g/m² de matière active (cires) en surface du produit.

Ce traitement de surface se fait par tout moyen classique, tel que pulvérisation au moyen de buses, enduction au moyen d'un cylindre, impression par rotogravure, ...

Afin de mieux distinguer la face traitée de la face non traitée, le produit selon l'invention peut présenter une différenciation visuelle des deux faces obtenues par exemple par une face marquée d'un motif et l'autre non marquée ou deux faces marquées de manière différente (avec des motifs différents) ou encore par coloration de l'une des faces, etc...

Les produits ainsi fabriqués, ayant des faces différenciées du fait du traitement de surface d'une des faces externes, sont ensuite découpés en formats et emballés dans des emballages souples ou sachets.

## Revendications

1. Tampon à démaquiller à base de fibres de coton hydrophiles, comprenant une face externe destinée à appliquer des produits aqueux sur la peau, **caractérisé en ce que** la face externe précitée comprend un agent retardant l'absorption des produits aqueux, le dit agent étant une émulsion de cire naturelle d'origine minérale, végétale ou animale.

2. Tampon selon la revendication 1, **caractérisé en ce que** la cire d'origine animale est de la cire de spermaceti et de préférence de la cire d'abeille.

3. Tampon selon la revendication 1, **caractérisé en ce que** la cire d'origine végétale est de la cire de candelilla ou de la cire de carnauba.

4. Tampon selon la revendication 1, **caractérisé en ce que** la cire d'origine minérale est de la cérésine ou de l'azocérite.

5. Tampon selon l'une des revendications précédentes, **caractérisé en ce que** l'agent est une émulsion comprenant au moins 30 % de matières actives.

6. Tampon selon la revendication 5, **caractérisé en ce que** la face destinée à appliquer des produits aqueux sur la peau comprend au moins 0,3 g/m² de matières actives.

7. Tampon selon l'une des revendications précédentes, **caractérisé en ce que** les produits aqueux sont des produits de soin tels que des produits cosmétiques comprenant notamment des produits de maquillage, des produits de démaquillage, des laits de toilette.

## Claims

1. Make-up removal pad based on hydrophilic cotton fibres, comprising an outer surface for the application of aqueous products to the skin, **characterised in that** the aforementioned outer surface comprises an agent which retards the absorption of the aqueous products, the said agent being a natural wax emulsion of animal, mineral or vegetable origin.

2. Pad according to claim 1, **characterised in that** the wax of animal origin is spermaceti wax or preferably beeswax.

3. Pad according to claim 1, **characterised in that** the wax of vegetable origin is candellila wax or carnauba wax.

4. Pad according to claim 1, **characterised in that** the wax of mineral origin is ceresin or azocerite.

5. Pad according to any one of the preceding claims, **characterised in that** the agent is an emulsion comprising at least 30% of active materials.

6. Pad according to claim 5, **characterised in that** the surface which is designed to apply aqueous products to the skin comprises at least 0.3 g/m² of active materials.

7. Pad according to any one of the preceding claims, **characterised in that** the aqueous products are care products such as cosmetic products comprising in particular make-up products, make-up removal products and toilet lotions.

## Patentansprüche

1. Bausch zum Abschminken auf Basis hydrophiler Baumwollfasern, umfassend eine äußere Seite, die dazu bestimmt ist, wässrige Produkte auf die Haut aufzutragen, **dadurch gekennzeichnet, dass** die zuvor erwähnte äußere Seite einen Wirkstoff umfasst, der die Absorption von wässrigen Produkten verzögert, wobei der Wirkstoff eine Naturwachsemulsion mineralischen, pflanzlichen oder tierischen Ursprungs ist.

2. Bausch nach Anspruch 1, **dadurch gekennzeichnet, dass** das Wachs tierischen Ursprungs Spermazet und vorzugsweise Bienenwachs ist.

3. Bausch nach Anspruch 1, **dadurch gekennzeichnet, dass** das Wachs pflanzlichen Ursprungs Kandelillawachs oder Karnaubawachs ist.

4. Bausch nach Anspruch 1, **dadurch gekennzeichnet, dass** das Wachs mineralischen Ursprungs Ceresin oder Azocerit ist.

5. Bausch nach einem der vorhergehenden Ansprüche, **dadurch gekennzeichnet, dass** der Wirkstoff eine Emulsion ist, die mindestens 30 % aktive Stoffe umfasst.

6. Bausch nach Anspruch 5, **dadurch gekennzeichnet, dass** die Seite, die dazu bestimmt ist, wässrige Produkte auf die Haut aufzutragen, mindestens 0,3 g/m² an aktiven Stoffen umfasst.

7. Bausch nach einem der vorhergehenden Ansprüche, **dadurch gekennzeichnet, dass** die wässrigen Produkte Pflegeprodukte sind, wie kosmetische Produkte, die insbesondere Schminkprodukte, Abschminkprodukte und Hautcremes umfassen.
